# FASCICULE DE BREVET EUROPEEN

(11) **EP 1 200 096 B1**
(45) Date de publication et mention de la délivrance du brevet: **16.06.2004**
(21) Numéro de dépôt: 00949689.4
(22) Date de dépôt: 07.07.2000
(51) Int. Cl.: A61K 31/57, A61K 47/00

(54) **SUSPENSION DE TIXOCORTOL PIVALATE, COLLUTOIRE A BASE DE CELLE-CI ET CONDITIONNEMENT LA CONTENANT**
TIXOCORTOLPIVALATHALTIGE PHARMAZEUTISCHE SUSPENSION,DARAUS HERGESTELLTES MUNDWASSER UND DIESE SUSPENSION ENTHALTENDE VERPACKUNG
TIXOCORTOL PIVALATE SUSPENSION, MOUTH-WASH BASED THEREON AND PACKAGING CONTAINING SAME

(30) Priorité: 22.07.1999 FR 9909556
(43) Date de publication de la demande: 02.05.2002
(73) Titulaire: Warner-Lambert Company LLC, Morris Plains, New Jersey 07950 (US)
(72) Inventeur: DOAT, Bernard, F-49000 Angers (FR)
(74) Mandataire: Hirsch, Denise
(86) Numéro de dépôt international: PCT/FR2000/001987
(87) Numéro de publication internationale: WO 2001/007053

(56) Documents cités:
- WO-A-95/11669
- WO-A-97/20578
- US-A- 4 877 781

## Description

La présente invention a pour objet une suspension de tixocortol pivalate, sous forme de collutoire et un récipient contenant cette suspension, le conditionnement comprenant de plus une pompe doseuse.

On connaît déjà plusieurs compositions à base de tixocortol (ou (11β)-11,17-dihydroxy-21-mercapto-pregn-4-ène-3,20-dione), notamment sous la forme du pivalate, pour le traitement local d'états inflammatoires, notamment des affections otorhinolaryngologiques. Ces compositions peuvent être nébulisées pour être dispensées au niveau de la gorge.

On connaît un conditionnement pressurisé équipé d'une valve doseuse et comprenant une suspension de pivalate de tixocortol dans du myristate d'isopropyle, le gaz propulseur étant le dichlorodifluorocarbone (F12, CFC). Le volume du collutoire émis est en moyenne de 180µl.

L'interdiction d'utiliser des CFC a entraîné la suppression de la valve doseuse associée au gaz propulseur. Le conditionnement a été remplacé par un conditionnement avec une pompe doseuse, la composition restant identique. Lors de l'utilisation, le patient comprime de l'air dans la chambre de la pompe, ce qui permet de délivrer environ 50µl de suspension.

Cette solution n'est cependant pas satisfaisante pour plusieurs raisons. Tout d'abord, la suspension ne permet pas de délivrer un volume de collutoire suffisant (50µl contre 180µl auparavant). Ensuite, cette suspension est relativement visqueuse, ce qui ne permet pas d'obtenir une bonne couverture de la zone à traiter. De plus, ce type de produit ne permet pas d'obtenir des gouttelettes de taille contrôlée qui ne passeront pas dans les bronches mais seront délivrées au niveau de la cavité bucco-pharyngée.

Il n'est pas possible d'ajuster le volume à 180 µl avec le myristate d'isopropyle, le goût étant inacceptable.

L'invention a donc pour but de fournir une suspension qui soit stable au stockage et qui soit facilement délivrable sous forme de collutoire ayant un volume et une dimension de gouttelettes appropriés.

Le document WO-A-9720578 décrit une suspension de pivalate de tixocortol dans un véhicule aqueux exempt d'agent de conservation, pour le traitement de pathologies oculaires et ophtalmiques.

Le document WO-A-9531964 décrit une composition de fluticasone propionate ayant une dimension particulaire inférieure à 12µm, en suspension dans une solution aqueuse comprenant un tensio-actif et un agent tampon, destinée à être administrée au niveau des bronches.

Le document WO-A-9511669 décrit une composition de d'étabonate de loteprednol en suspension dans une solution aqueuse comprenant un polymère et un tensio-actif non ionique et un agent d'isotonicité (glycérol).

Aucun des documents ci-dessus n'enseigne ni ne suggère la présente invention.

Ainsi, l'invention fournit une suspension aqueuse de pivalate de tixocortol, comprenant:
(a) de 0,05 à 10%m/v de pivalate de tixocortol en suspension, présentant une dimension particulaire telle que sa taille moyenne D50 est comprise entre 0,5 et 20µm;
(b) de 0,005 à 2,5%m/v d'un tensio-actif;
(c) de 1 à 50%m/v d'un agent de viscosité, de sorte que la suspension présente une viscosité comprise entre 1,2 et 2 mPa.s; et
(d) la balance en eau et adjuvants classiques

L'invention fournit aussi un collutoire à base de la suspension définie ci-dessus, et présentant un volume compris entre 100 et 300µl.

L'invention fournit aussi un conditionnement comprenant un récipient contenant la suspension définie ci-dessus, muni d'une pompe doseuse.

Enfin, l'invention fournit un procédé de préparation de la suspension définie ci-dessus, comprenant les étapes suivantes:
(1) préparation de la solution aqueuse avec la balance d'eau, le tensio-actif, l'agent de viscosité, éventuellement le second principe actif;
(2) mise en suspension du pivalate de tixocortol dans ladite solution.

L'invention est maintenant décrite plus en détail dans la description qui suit.

### Pivalate de tixocortol

Le principe actif est sous forme micronisée, et il présente une dimension particulaire telle que sa taille moyenne D50 (D50, taille sous laquelle se situent 50% en volume des particules) est comprise entre 0,5 et 20µm, de préférence entre 1 et 10µm. Par exemple, la taille des particules pourra être telle que la valeur de D90 (D90, taille sous laquelle se situent 90% en volume des particules) est inférieure à 10µm.

Le principe actif est préparé par des méthodes classiques. Les cristaux sont réduits à la taille souhaitée par des méthodes elles aussi classiques, par exemple micronisation.

Le principe actif sous forme micronisée est aussi disponible dans lé commerce, par exemple chez PPG (Avrillé, France).

La suspension selon l'invention comprendra, en poids par rapport au volume final de la suspension (en %m/v, c'est-à-dire en gramme pour 100ml de suspension) par exemple de 0,05 à 10%m/v, notamment de 0,1 à 5%m/v, avantageusement de 0,1 à 0,5%m/v, de pivalate de tixocortol.

### Second principe actif

La suspension comprend avantageusement un second principe actif, notamment un antiseptique. Cet antiseptique peut être de la chlorhexidine (notamment sous forme saline). De préférence l'antiseptique est le digluconate de chlorhexidine.

De préférence, le second principe actif est soluble dans la solution aqueuse.

Le digluconate de chlorhexidine est disponible sous forme de solution aqueuse, par exemple dosée à 20%m/v.

La suspension selon l'invention comprendra, en poids par rapport au volume final de la suspension (en %m/v) par exemple de 0,05 à 10%m/v, notamment de 0,1 à 5%m/v, avantageusement de 0,1 à 0,5%m/v de digluconate de chlorhexidine (exprimé sous forme sèche).

D'autres principes actifs peuvent aussi être présents.

### Tensio-actif, agent mouillant

Le tensio-actif est un agent mouillant qui peut être sélectionné parmi les tensio-actifs pharmaceutiquement acceptables. A titre d'exemple, on peut citer les trioléate, mono-oléate, monolaurate de sorbitan, ou leurs dérivés polyoxyéthylénés (polysorbate 20, 40, 60, 80); lécithine naturelle ou de synthèse; éthers gras polyoxyéthylénés d'oléyle, de stéaryle, de lauryle; copolymères blocs polyoxyéthylène-polyoxypropylène; dioléate de diéthylène glycol; oléate de tétrahydrofurfuryle, oléate d'éthyle, mono-oléate de glycéryl, monolaurate de glycéryl; PEG400 à PEG4000; et dérivés d'ammonium quaternaire. Ces tensio-actifs sont disponibles dans le commerce, par exemple sous les dénominations Pluronic, Poloxamer, Tween. Les tensio-actifs agents mouillants préférés dans l'invention sont les dérivés d'ammonium quaternaire, notamment les dérivés cycliques comprenant l'atome d'azote quaternisé dans le cycle ou non. A titre d'exemple, on peut citer le picolinium, le benzalkonium et le cétylpyridinium, ce dernier étant préféré. Le contre-ion peut être n'importe quel contre-ion classique, par exemple le chlorure. Des mélanges sont aussi appropriés.

La suspension selon l'invention comprendra, en masse par rapport au volume final de la suspension (en %m/v) par exemple de 0,005 à 2,5%m/v, notamment de 0,01 à 0,5%m/v, avantageusement de 0,01 à 0,05%m/v de tensio-actif.

### Agent de viscosité

L'agent de viscosité a pour objet d'ajuster la viscosité de la solution et in fine de la suspension. Cet agent de viscosité (ou agent épaississant) peut être choisi parmi les polymères hydrosolubles (tels que polyvinylpyrrolidone PVP, polyvinylalcohol PVA; les dérivés de cellulose tels que hydroxypropylmethylcellulose HPMC, hydroxymethylcellulose HMC, hydroxyethylcellulose HEC, hydroxypropylcellulose HPC, carboxymethylcellulose CMC; (cyclo)dextrine, etc.) et les polyols et leurs dérivés. Cette dernière classe comprend par exemple le glycérol, le sorbitol et le maltitol. Cette dernière classe est préférée, et on préférera plus particulièrement le glycérol. Celui-ci présente en outre des propriétés édulcorantes et adoucissantes (émollientes). Des mélanges sont appropriés.

On préférera une solution qui est relativement peu visqueuse. La viscosité de la suspension, mesurée selon la méthode du tube capillaire (Ph. Eur. 2-2-9) est avantageusement comprise entre 1,2 et 2 mPa.s, de préférence entre 1,4 et 1,8 mPa.s (la viscosité de l'eau pure étant de 1,03 mPa.s).

La suspension légèrement visqueuse permet d'obtenir une meilleure couverture de la zone à traiter, et une meilleure adhérence au niveau des voies aériennes supérieures. De plus, cette viscosité permet de ralentir la sédimentation des particules de principe actif.

La suspension selon l'invention comprendra, en poids par rapport au volume final de la suspension (en %m/v) par exemple de 1 à 50%m/v, notamment de 5 à 30%m/v, avantageusement de 10 à 20%m/v d'agent de viscosité.

### Adjuvants

Les adjuvants classiques peuvent être ajoutés dans la composition. Parmi ces adjuvants, on peut citer les agents édulcorants, les arômes, les tampons, les agents conservateurs, etc. Ces adjuvants, ainsi que leurs quantités, sont classiques et connus de l'homme de l'art.

En tant qu'agent édulcorant, on peut utiliser notamment l'aspartame, le saccharinate de sodium, l'acésulfame de potassium, le cyclamate de sodium, ou le glycyrrhizinate d'ammonium. On préférera l'acésulfame de potassium. La teneur en agent édulcorant est par exemple de 0,025 à 0,5%m/v, notamment de 0,025 à 0,05%m/v, avantageusement de 0,05 à 0,25%m/v d'agent édulcorant. L'agent de sapidité (arôme) peut être présent en solution ou en suspension. Des mélanges sont appropriés.

### Suspension

Cette suspension présente de bonnes propriétés en ce qui concerne:
- la vitesse de sédimentation (stabilité)
- évolution de la taille et de la forme des particules de pivalate de tixocortol sous l'effet des cycles de température.
- faculté de remise en suspension.

En ce qui concerne la vitesse de sédimentation, le taux de sédimentation à température ambiante est supérieur à 90% après 45 min, c'est-à-dire que la hauteur du front de sédimentation est de 90% de la hauteur totale initiale.

En ce qui concerne l'évolution de la taille et de la forme des particules de pivalate de tixocortol sous l'effet des cycles de température, on ne note aucune modification. Après un cycle 50°C pendant 1 h, 5°C pendant 1 h, 50°C pendant 1 h, 5°C pendant 1 h, 50°C pendant 1 h, il n'y a aucune différence avec un échantillon témoin stocké à température ambiante.

En ce qui concerne la faculté de remise en suspension, la présente invention offre des résultats excellents. Après 24 h au repos à température ambiante, il suffit de 2 à 4 retournements de l'éprouvette pour que la préparation se remette en suspension homogène. Ainsi, l'utilisateur n'aura pas à agiter le flacon pendant plusieurs minutes avant emploi, mais quelques retournements du flacon suffiront.

La suspension comprend le principe actif toujours sous forme micronisée, l'aggrégation ou flocculation étant minime. Ainsi, la taille des particules de principe actif, en suspension, est en général au maximum de cinq fois, de préférence deux fois, la taille initiale.

La suspension selon l'invention présente une bonne résistance à la contamination microbienne.

La suspension est préparée par tout procédé approprié. Un procédé typique comprend les étapes suivantes, par exemple mises en oeuvre à température ambiante:
- préparation d'une solution aqueuse avec l'eau, l'agent antiseptique (digluconate de chlorhexidine), le tensio-actif (cétylpyridinium), les adjuvants;
- solubilisation/dispersion de l'agent de viscosité (glycérol); et
- mise en suspension (dispersion puis homogénéisation) du pivalate de tixocortol.

On peut aussi utiliser un procédé en deux étapes, la première étape comprenant la préparation de la solution aqueuse avec tous les ingrédients hydrosolubles ou hydrodispersibles, puis la mise en suspension du pivalate de tixocortol.

Le matériel utilisé est classique et connu de l'homme du métier.

### Collutoire

Le collutoire se présente sous forme d'un aérosol de gouttelettes.

Le volume dispensé du collutoire est en général compris entre 100 et 300µl, de préférence entre 150 et 220µl, avantageusement environ 180µl.

Les gouttelettes présentent une dimension moyenne (D'50) telle que ces gouttelettes ne pénètrent pas dans les bronches, mais au contraire restent au niveau des zones de la gorge destinées à recevoir le traitement anti-inflammatoire, à savoir la zone laryngée et rhinopharyngée. Il convient de noter qu'en général la température ne modifie pas la granulométrie de la pulvérisation. D'50 est en général compris entre 20 et 500µm, avantageusement entre 50 et 200µm, de préférence environ 100µm.

La suspension et le collutoire à base de celle-ci sont destinés notamment au traitement des inflammations de la zone laryngée, bucco pharyngée et rhinopharyngée, en particulier angine, laryngites et rhinopharyngites. Une dose comprendra en général une quantité de pivalate de tixocortol comprise entre 0,2 et 1,2 mg, en particulier environ 0,6 mg. Une dose comprendra en général une quantité de digluconate de chlorhexidine comprise entre 0,05 et 0,6 mg, en particulier environ 0,18 mg.

### Conditionnement

Le conditionnement comprend un récipient et une pompe doseuse.

La pompe doseuse est adaptée pour délivrer des volumes appropriés de gouttelettes de suspension sous forme de collutoire. Une telle pompe doseuse est disponible dans le commerce.

Les exemples suivants illustrent l'invention sans la limiter.

### Exemple 1.

On prépare une composition ayant la composition centésimale (en m/v) suivante:

| Ingrédient | Quantité |
|---|---|
| Pivalate de tixocortol | 0,333 g |
| Digluconate de chlorhexidine | 0,143 g |
| Polysorbate 80 | 0,050 g |
| Glycérol | 15 g |
| Adjuvants (arôme et édulcorant) | 0,7 g |
| Eau | qsp 100 ml |

Dans un premier temps, on prépare une solution aqueuse avec l'eau, la solution à 20%m/v de digluconate de chlorhexidine, le polysorbate 80, les adjuvants, par mélange à température ambiante.

Dans un second temps on disperse le glycérol.

Enfin, on met en suspension le pivalate de tixocortol par dispersion puis homogénéisation.

### Exemple 2.

On prépare une composition ayant la composition centésimale (en m/v) suivante:

| Ingrédient | Quantité |
|---|---|
| Pivalate de tixocortol | 0,333 g |
| Digluconate de chlorhexidine | 0,143 g |
| Chlorure de cétylpyridinium | 0,025 g |
| Glycérol | 15 g |
| Adjuvants (arôme et édulcorant) | 0,7 g |
| Eau | qsp 100 ml |

On procède de la même façon que dans l'exemple 1 mais en remplaçant le polysorbate 80 par du chlorure de cétylpyridinium.

### Exemple 3.

On remplit un récipient avec les suspensions des exemples 1 ou 2, puis on sertit une pompe doseuse sur le corps du récipient. La dose délivrée est de 180µl de collutoire, correspondant à 0,6 mg et 0,257mg de pivalate de tixocortol et de digluconate de chlorhexidine, respectivement. Les gouttelettes du collutoire présentent une taille d'environ 100µm.

## Revendications

1. Suspension aqueuse de pivalate de tixocortol, comprenant:
(a) de 0,05 à 10%m/v de pivalate de tixocortol en suspension, présentant une dimension particulaire telle que sa taille moyenne D50 est comprise entre 0,5 et 20µm;
(b) de 0,005 à 2,5%m/v d'un tensio-actif;
(c) de 1 à 50%m/v d'un agent de viscosité, de sorte que la suspension présente une viscosité comprise entre 1,2 et 2 mPa.s; et
(d) la balance en eau et adjuvants classiques

2. La suspension selon la revendication 1, dans laquelle le pivalate de tixocortol présente une dimension particulaire telle que sa taille moyenne D50 est comprise entre 1 et 10µm.

3. La suspension selon la revendication 1 ou 2, dans laquelle le pivalate de tixocortol présente une dimension particulaire telle que sa taille moyenne D90 est inférieure à 10µm.

4. La suspension selon l'une quelconque des revendication 1 à 3, dans laquelle le tensio-actif est dérivé d'ammonium quaternaire.

5. La suspension selon la revendication 4, dans laquelle le dérivé d'ammonium quaternaire est du cétylpyridinium.

6. La suspension selon l'une quelconque des revendications 1 à 5, dans laquelle l'agent de viscosité est le glycérol.

7. La suspension selon l'une quelconque des revendications 1 à 7, comprenant, en % en masse par volume de la suspension:
(a) de 0,1 à 5%m/v de pivalate de tixocortol;
(b) de 0,01 à 0,5%m/v d'un tensio-actif;
(c) de 5 à 30%m/v d'un agent de viscosité; et
(d) la balance en eau et adjuvants classiques.

8. La suspension selon l'une quelconque des revendications 1 à 7, comprenant, en % en masse par volume de la suspension:
(a) de 0,1 à 0,5%m/v de pivalate de tixocortol;
(b) de 0,01 à 0,05%m/v d'un tensio-actif;
(c) de 10 à 20%m/v d'un agent de viscosité; et
(d) la balance en eau et adjuvants classiques.

9. La suspension selon l'une quelconque des revendications 1 à 8, qui présente une viscosité comprise entre 1,4 et 1,8 mPa.s.

10. La suspension selon l'une quelconque des revendications 1 à 9, comprenant, en outre:
(e) un antiseptique.

11. La suspension selon la revendication 10, dans laquelle l'antiseptique est la chlorhexidine.

12. La suspension selon la revendication 10 ou 11, comprenant, en % en masse par volume de la suspension:
(e) de 0,05 à 10%m/v d'antiseptique.

13. La suspension selon la revendication 10, 11 ou 12, comprenant, en % en masse par volume de la suspension:
(e) de 0,1 à 5%m/v d'antiseptique.

14. La suspension selon la revendication 1 ayant la composition centésimale suivante : 0,333g de pivalate de tixocortol, 0,143g de digluconate de chlorhexidine, 0,025g de chlorure de cétylpyridinium, 15g de glycérol, 0,7g d'adjuvants et la quantité d'eau nécessaire pour atteindre un volume de 100ml

15. La suspension selon l'une quelconque des revendications 1 à 14, pour utilisation dans le traitement des inflammations de la zone laryngée, bucco pharyngée et rhinopharyngée.

16. Collutoire à base d'une suspension selon l'une quelconque des revendications 1 à 15, présentant un volume délivré compris entre 100 et 300µl.

17. Collutoire selon la revendication 16, présentant un volume délivré compris entre 150 et 220µl.

18. Le collutoire selon la revendication 16 ou 17, dont les gouttelettes présentent une dimension particulaire telle que sa taille moyenne D'50 est comprise entre 20 et 500µm.

19. Le collutoire selon la revendication 16, 17 ou 18, dont les gouttelettes présentent une dimension particulaire telle que sa taille moyenne D'50 est comprise entre 50 et 200µm.

20. Le collutoire selon l'une quelconque des revendications 16 à 19, comprenant entre 0,2 et 1,2 mg de pivalate de tixocortol.

21. Conditionnement comprenant un récipient contenant la suspension selon l'une quelconque des revendications 1 à 15, muni d'une pompe doseuse.

22. Procédé de préparation de la suspension selon l'une quelconque des revendications 1 à 15, comprenant les étapes suivantes:
(1) préparation de la solution aqueuse avec la balance d'eau, le tensio-actif, l'agent de viscosité, éventuellement le second principe actif;
(2) mise en suspension du pivalate de tixocortol dans ladite solution.

23. Procédé selon la revendication 22, dans lequel l'étape (1) comprend deux sous-étapes, la première sous-étape comprenant la préparation d'une solution aqueuse avec la balance d'eau, le tensio-actif, et éventuellement le second principe actif, la seconde sous-étape comprenant la solubilisation/dispersion de l'agent de viscosité.

24. Procédé de préparation d'un collutoire selon l'une quelconque des revendications 16 à 20, comprenant l'action de la pompe doseuse du conditionnement selon la revendication 21.

## Patentansprüche

1. Tixocortolpivalathaltige wässrige Suspension, die umfasst:
(a) 0,05 bis 10 % m/v Tixocortolpivalat in Suspension, das eine Partikeldimension aufweist, so dass seine mittlere Korngröße D50 zwischen 0,5 und 20 µm beträgt,
(b) 0,005 bis 2,5 % m/v eines Tensids,
(c) 1 bis 50 % m/v eines Viskositätsmittels, so dass die Suspension eine Viskosität aufweist, die zwischen 1,2 und 2 mPa.s beträgt und
(d) den Ausgleich an Wasser und klassischen Hilfsmittel.

2. Suspension nach Anspruch 1, wobei das Tixocortolpivalat eine Partikeldimension aufweist, so dass seine mittlere Korngröße D50 zwischen 1 und 10 µm beträgt.

3. Suspension nach Anspruch 1 oder 2, wobei das Tixocortolpivalat eine Partikeldimension aufweist, so dass seine mittlere Größe D90 unter 10 µm liegt.

4. Suspension nach irgendeinem der Ansprüche 1 bis 3, wobei das Tensid von quartärem Ammonium abgeleitet ist.

5. Suspension nach Anspruch 4, wobei das Derivat des quartären Ammoniums Ketylpyridinium ist.

6. Suspension nach irgendeinem der Ansprüche 1 bis 5, wobei das Viskositätsmittel Glyzerin ist.

7. Suspension nach irgendeinem der Ansprüche 1 bis 6, welche in % der Masse pro Volumen der Suspension umfasst:
(a) 0,1 bis 5 % m/v Tixocortolpivalat,
(b) 0,01 bis 0,5 % m/v eines Tensids,
(c) 5 bis 30 % m/v eines Viskositätsmittels und
(d) den Ausgleich an Wasser und klassischen Hilfsmittel.

8. Suspension nach irgendeinem der Ansprüche 1 bis 7, welche in % der Masse pro Volumen der Suspension umfasst:
(a) 0,1 bis 0,5 % m/v Tixocortolpivalat,
(b) 0,01 bis 0,05 % m/v eines Tensids,
(c) 10 bis 20 % m/v eines Viskositätsmittels und
(d) den Ausgleich an Wasser und klassischen Hilfsmittel.

9. Suspension nach irgendeinem der Ansprüche 1 bis 8, welche eine Viskosität aufweist, die zwischen 1,4 und 1,8 mPa.s liegt.

10. Suspension nach irgendeinem der Ansprüche 1 bis 9, welche außerdem umfasst:
(e) ein Antiseptikum.

11. Suspension nach Anspruch 10, wobei das Antiseptikum Chlorhexidin ist.

12. Suspension nach Anspruch 10 oder 11, welche in % Masse pro Volumen der Suspension umfasst
(e) 0,05 bis 10 % m/v Antiseptikum.

13. Suspension nach Anspruch 10, 11 oder 12, welche in % Masse pro Volumen der Suspension umfasst
(e) 0,1 bis 5 % m/v Antiseptikum.

14. Suspension nach Anspruch 1, welche die folgende hundertteilige Zusammensetzung aufweist: 0,333 g Tixocortolpivalat, 0,143 g Chlorhexidin-Diglukonat, 0,025 g Ketylpyridiniumchiorid, 15 g Glycerin, 0,7 g Hilfsmittel und die notwendige Menge an Wasser, um ein Volumen von 100 ml zu erhalten.

15. Suspension nach irgendeinem der Ansprüche 1 bis 14 zur Anwendung bei der Behandlung von Entzündungen im Kehlkopf-, Mundrachensowie Nasenrachenraum.

16. Mundwasser auf der Grundlage einer Suspension nach irgendeinem der Ansprüche 1 bis 15, das ein abgegebenes Volumen zwischen 100 und 300 µl aufweist.

17. Mundwasser nach Anspruch 16, das ein abgegebenes Volumen zwischen 150 und 220 µl aufweist.

18. Mundwasser nach Anspruch 16 oder 17, dessen Tröpfchen eine Partikeldimension aufweisen, so dass seine mittlere Korngröße D'50 zwischen 20 und 500 µm beträgt.

19. Mundwasser nach Anspruch 16, 17 oder 18, dessen Tröpfchen eine Partikeldimension aufweisen, so dass seine mittlere Korngröße D'50 zwischen 50 und 200 µm beträgt.

20. Mundwasser nach irgendeinem der Ansprüche 16 bis 19, welches zwischen 0,2 und 1,2 mg Tixocortolpivalat enthält.

21. Verpackung, die einen Behälter umfasst, der die Suspension nach irgendeinem der Ansprüche 1 bis 15 enthält und mit einer Dosierpumpe versehen ist.

22. Verfahren zur Zubereitung der Suspension nach irgendeinem der Ansprüche 1 bis 15, das die folgenden Verfahrensschritte umfasst:
(1) Zubereitung der wässrigen Lösung mit dem Wasseranteil, dem Tensid, dem Viskositätsmittel, eventuell einem zweiten Wirkstoff,
(2) Suspension des Tixocortolpivalats in dieser Lösung.

23. Verfahren nach Anspruch 22, wobei der Schritt (1) aus zwei Unterschritten besteht, wobei der erste Unterschritt der Zubereitung einer wässrigen Lösung mit dem Wasseranteil, dem Tensid, eventuell einem zweiten Wirkstoff und der zweite Unterschritt die Solubilisation/Dispersion des Viskositätsmittels umfasst.

24. Verfahren zur Zubereitung eines Mundwassers nach irgendeinem der Ansprüche 16 bis 20, das die Funktion der Dosierpumpe der Verpackung nach Anspruch 21 umfasst.

## Claims

1. An aqueous suspension of tixocortol pivalate, comprising:
(a) from 0.05 to 10% m/v of suspended tixocortol pivalate, having a particle dimension such that its average size D50 is between 0.5 and 20 µm;
(b) from 0.005 to 2.5% m/v of a surfactant;
(c) from 1 to 50% m/v of a viscosity agent so that the suspension has a viscosity between 1.2 and 2 mPa.s; and
(d) the balance being water and conventional additives.

2. The suspension according to claim 1, wherein tixocortol pivalate has a particle dimension such that its average size D50 is between 1 and 10 µm.

3. The suspension according to claim 1 or 2, wherein the tixocortol pivalate has a particle dimension such that its average size D90 is less than 10 µm.

4. The suspension according to any of claims 1 to 3, wherein the surfactant is derived from quaternary ammonium.

5. The suspension according to claim 4, wherein the quaternary ammonium derivative is cetylpyridinium.

6. The suspension according to any of claims 1 to 5, wherein the viscosity agent is glycerol.

7. The suspension according to any of claims 1 to 6, comprising in mass % per volume of the suspension:
(a) from 0.1 to 5% m/v of tixocortol pivalate,
(b) from 0.01 to 0.5% m/v of a surfactant;
(c) from 5 to 30% m/v of a viscosity agent; and
(d) the balance being water and conventional additives.

8. The suspension according to any of claims 1 to 7, comprising in mass % per volume of the suspension:
(a) from 0.1 to 0.5% m/v of tixocortol pivalate,
(b) from 0.01 to 0.05% m/v of a surfactant;
(c) from 10 to 20% m/v of a viscosity agent; and
(d) the balance being water and conventional additives.

9. The suspension according to any of claims 1 to 8, which has a viscosity between 1.4 and 1.8 mPa.s.

10. The suspension according to any of claims 1 to 9, further comprising:
(e) an antiseptic.

11. The suspension according to claim 10, wherein the antiseptic is chlorhexidine.

12. The suspension according to claim 10 or 11, comprising in mass % per volume of suspension:
(e) from 0.05 to 10% m/v of antiseptic.

13. The suspension according to claim 10, 11 or 12, comprising in mass % per volume of suspension:
(e) from 0.1 to 5% m/v of antiseptic.

14. The suspension according to claim 1 with the following centesimal composition: 0.333 g of tixocortol pivalate, 0.143 g of chlorhexidine digluconate, 0.025 g of cetylpyridinium chloride, 15 g of glycerol, 0.7 g of additives and the amount of water required to reach a volume of 100 ml.

15. The suspension according to any of claims 1 to 14, for use in the treatment of inflammations of the laryngeal, buccopharyngeal and rhinopharyngeal area.

16. A collutory based on a suspension according to any of claims 1 to 15, having a delivered volume between 100 and 300 µl.

17. The collutory according to claim 16, having a delivered volume between 150 and 220 µl.

18. The collutory according to claim 16 or 17, with droplets having a particle dimension such that its average size D'50 is between 20 and 500 µm.

19. The collutory according to claim 16, 17 or 18, with droplets having a particle dimension such that its average size D'50 is between 50 and 200 µm.

20. The collutory according to any of claims 16 to 19, comprising between 0.2 and 1.2 mg of tixocortol pivalate.

21. A packaging comprising a container containing the suspension according to any of claims 1 to 15, provided with metering pump.

22. A method for preparing the suspension according to any of claims 1 to 15, comprising the following steps:
(1) preparation of the aqueous solution with the water balance, the surfactant, the viscosity agent, optionally the second active ingredient;
(2) suspension of tixocortol pivalate in said solution.

23. The method according to claim 22, wherein step (1) comprises two sub-steps, the first sub-step comprising the preparation of an aqueous solution with the water balance, the surfactant and optionally the second active ingredient, the second sub-step comprising the solubilization/dispersion of the viscosity agent.

24. A method for preparing a collutory according to any of claims 16 or 20, comprising the action of the metering pump of the packaging according to claim 21.
